(19) Europäisches Patentamt
European Patent Office
Office européen des brevets

(11) EP 3 155 588 B1

(12) EUROPÄISCHE PATENTSCHRIFT

(45) Veröffentlichungstag und Bekanntmachung des Hinweises auf die Patenterteilung:
15.01.2020  Patentblatt 2020/03

(51) Int Cl.:
G06T 7/00 (2017.01)     G06T 7/11 (2017.01)
G06T 7/174 (2017.01)    A61B 5/00 (2006.01)

(21) Anmeldenummer: 15721135.0

(22) Anmeldetag: 16.04.2015

(86) Internationale Anmeldenummer:
PCT/EP2015/058260

(87) Internationale Veröffentlichungsnummer:
WO 2015/188964 (17.12.2015 Gazette 2015/50)

(54) GANZKÖRPERBILDAUFNAHME- UND BILDVERARBEITUNGSSYSTEM SOWIE VERFAHREN ZU DESSEN BETRIEB

WHOLE-BODY IMAGE RECORDING AND IMAGE PROCESSING SYSTEM AND METHOD FOR OPERATING SAME

SYSTÈME D'ACQUISITION ET DE TRAITEMENT D'UNE IMAGE D'UN CORPS COMPLET ET PROCÉDÉ POUR FAIRE FONCTIONNER CELUI-CI

(84) Benannte Vertragsstaaten:
AL AT BE BG CH CY CZ DE DK EE ES FI FR GB GR HR HU IE IS IT LI LT LU LV MC MK MT NL NO PL PT RO RS SE SI SK SM TR

(30) Priorität: 13.06.2014  DE 102014108357

(43) Veröffentlichungstag der Anmeldung:
19.04.2017  Patentblatt 2017/16

(73) Patentinhaber: Fotofinder Systems GmbH
84364 Bad Birnbach (DE)

(72) Erfinder: MAYER, Andreas
94032 Passau (DE)

(74) Vertreter: advotec.
Patent- und Rechtsanwälte
Widenmayerstrasse 4
80538 München (DE)

(56) Entgegenhaltungen:
WO-A1-2004/095372     WO-A1-2012/064170
WO-A2-2006/078902     WO-A2-2007/037848
US-A1- 2009 281 420    US-A1- 2010 272 333

EP 3 155 588 B1

**Beschreibung**

Die Erfindung betrifft ein Ganzkörperbildaufnahme- und Bildverarbeitungssystem sowie ein Verfahren zum Betrieb eines solchen Systems. Im Rahmen der Behandlung von Schuppenflechte (Psoriasis) ist es bekannt, einen so genannten "Psoriasis Area and Severity Index" (PASI) zu erstellen und anhand dieses Indexes den Krankheitsverlauf bzw. den Behandlungsverlauf zu dokumentieren. Der PASI wurde dabei bisher nach einer Begutachtung der Hautoberfläche eines Patienten händisch bzw. als Überschlag oder im Rahmen einer Abschätzung ermittelt. Diese Ermittlung hat jedoch den Nachteil, dass sie nur von hochspezialisiertem medizinisch geschultem und erfahrenem Fachpersonal durchgeführt werden kann. Weiter ist die Bestimmung des PASI aufgrund der benötigten Zeit zur Begutachtung der gesamten Hautoberfläche überaus zeitintensiv. Schließlich ist der wohl gravierendste Nachteil an der bisherigen Bestimmung des PASI darin zu sehen, dass zwar grundlegende Kriterien und ein Versuch zur Standardisierung des PASI vorhanden sind, diese jedoch von jedem Anwender und selbst von Patient zu Patient unterschiedlich oder gar von Untersuchung zu Untersuchung unterschiedlich angewendet werden. Damit sinken die Vergleichbarkeit der ermittelten Werte, die Reproduzierbarkeit der ermittelten Werte und allgemein dementsprechend auch die gesamte Aussagekraft der ermittelten Werte des PASI erheblich.

Die WO 2012/064170 A1 offenbart ein Verfahren zum Betrieb eines Ganzkörperbildaufnahmesystems aufweisend ein automatisches Abbildungssystem, eine Speichereinrichtung sowie eine Recheneinrichtung umfassend eine Bildverarbeitungsvorrichtung, eine Auswerteeinrichtung sowie eine Ausgabeeinheit und eine Eingabeeinheit. Das Verfahren sieht die Bewertung von Daten aus digitalen Bildern von Schuppenflechte zur Bewertung der Schuppigkeit unter Verwendung des Aufnahmesystems vor, um insbesondere den PASI-Wert zu erhalten.

Die WO 2004/095372 A1 offenbart ein Verfahren zum Ermitteln und Bewerten von Hautwunden unter Erfassung eines Gesamtkörperstatus und Farbbewertung der betroffenen Hautregion.

Es ist daher die Aufgabe der vorliegenden Erfindung, ein System sowie ein Verfahren zum Betrieb eines solchen Systems vorzuschlagen, das einerseits die Ermittlung des PASI weitestgehend automatisiert und damit die benötigte Zeit drastisch reduziert und zugleich die Erstellung des PASI hochgradig objektiviert und damit den Aussagegehalt bzw. die Reproduzierbarkeit stark verbessert.

Diese Aufgabe wird durch ein erfindungsgemäßes Verfahren gemäß Anspruch 1 sowie durch eine erfindungsgemäße Vorrichtung gemäß Anspruch 12 gelöst. Vorteilhafte Ausführungen des Verfahrens sowie der Vorrichtung sind Gegenstand der Unteransprüche.

Das erfindungsgemäße Verfahren dient zum Betrieb eines Ganzkörperbildaufnahme-und Bildverarbeitungssystems, welches zumindest ein Abbildungssystem, eine Speichereinrichtung sowie eine Recheneinrichtung umfassend eine Bildverarbeitungsvorrichtung, eine Auswerteeinrichtung sowie eine Ausgabe- und eine Eingabeeinheit des Systems aufweist.

Dabei sieht das erfindungsgemäße Verfahren folgende Verfahrensschritte vor:
Zunächst erfolgt die Aufnahme digitaler Bilder eines Untersuchungskörpers anhand einer Mehrzahl vorgegebener Positionierungen des Untersuchungskörpers bezüglich des Abbildungssystems durch das Abbildungssystem.

In einem weiteren Verfahrensschritt erfolgt die Erstellung von zumindest einem Kombinationsbild aus zumindest zwei unterschiedlichen Bildern der gleichen Positionierung des Untersuchungskörpers mittels der Bildverarbeitungsvorrichtung. Dabei kann vorgesehen sein, dass die vom Abbildungssystem erzeugten Bilder des Untersuchungskörpers zur Speicherung an die Speichereinrichtung übertragen werden und die Recheneinrichtung, insbesondere die Bildverarbeitungsvorrichtung auf den Inhalt der Speichereinrichtung zurückgreifen, also die mit dem Abbildungssystem erzeugten Bilder abrufen und entsprechend verarbeiten kann.

In einem weiteren Schritt des Verfahrens erfolgt die Identifizierung von der Hautoberfläche des Untersuchungskörpers in den Bildern und/oder Kombinationsbildern mittels der Bildbearbeitungsvorrichtung. Dazu kann beispielsweise vorgesehen sein, dass ein aufgenommenes Bild und/oder Kombinationsbild in das YCbCr-Farbmodell überführt wird, anschließend der Cr-Anteil, also der Red-Green-Crominenz-Anteil des Bildes bzw. Kombinationsbildes herausgefiltert wird und anschließend die Hautoberfläche anhand eines ETP-Algorithmus identifiziert wird. Als Ergebnis werden also in den Bildern bzw. Kombinationsbildern Pixel über ihre jeweilige Position bzw. Adressierung identifiziert, die die Hautoberfläche des Untersuchungskörpers in den Bildern bzw. Kombinationsbildern abbilden.

In einem weiteren Verfahrensschritt ist vorgesehen, dass für die in den Bildern bzw. Kombinationsbildern identifizierten Hautoberflächen im Vorfeld vordefinierte Segmente identifiziert werden. Dies bedeutet mit anderen Worten ausgedrückt, dass verschiedene Teile identifizierter Hautoberfläche in unterschiedlichen Bildern bzw. Kombinationsbildern zusammengefasst werden, da sie ein identisches anatomisches Segment des Untersuchungskörpers aus unterschiedlichen Perspektiven bzw. aus unterschiedlichen Positionierungen des Untersuchungskörpers zum Abbildungssystem abbilden. Es ist also vorgesehen, dass die gesamte identifizierte Hautoberfläche in Bildern bzw. in den Kombinationsbildern je einem Segment zugewiesen wird. Es werden also Pixel aus verschiedenen Bildern bzw. Kombinationsbildern ein und demselben Segment zugeordnet. Damit wir die Berechnung des PASI insgesamt auf anatomische Segmente statt auf die einzelnen Bilder oder Kombinationsbilder gestützt. Den Verfahrensschritt der Segmentierung kann die Bildverarbeitungsvorrichtung anhand allgemein bekannter Formerkennungsalgorithmen vollziehen.

In einem weiteren Verfahrensschritt erfolgt die Identifizierung von Hautregionen der identifizierten Hautoberfläche in den Bildern bzw. Kombinationsbildern, die Rötung oder Schorfbildung bzw. Plaquebildung aufweisen. Dies bedeutet, dass insgesamt die Läsionen auf der Hautoberfläche identifiziert werden, wobei die Eigenschaften Rötung und Schorfbildung zur Identifizierung herangezogen werden. Die Identifizierung erfolgt abermals mittels der Bilderverarbeitungsvorrichtung zur Feststellung bzw. zur Identifizierung von Hautregionen in den Bildern bzw. Kombinationsbildern, die Plaque bzw.

Schorf aufweisen. Die Bilder können dabei derart gefiltert bzw. bearbeitet werden, dass farblose bzw. weiße Bereiche der Bilder hervorgehoben bzw. herausgefiltert werden. Dies kann beispielsweise derart erfolgen, dass die Bilder in den HSV-Bildstandard übertragen und anschließend lediglich der S- und der V-Kanal des Bildes bzw. der Bilder berücksichtigt werden. Wie oben bereits angeführt, führt die Identifizierung in den einzelnen Bildern und Kombinationsbildern durch die vorangegangene Segmentierung der Bilder oder Kombinationsbilder gleichzeitig zu einer Identifizierung im Bezug auf die einzelnen Segmente.

In einem weiteren Verfahrensschritt erfolgt die Berechnung der anteiligen Fläche der identifizierten Hautregion je Segment mittels der Auswerteeinrichtung. Dazu werden jeweils die als gerötet oder verschorft identifizierten Hautregionen der identifizierten Hautoberfläche, die einem bestimmten Segment zugeordnet sind, aus unterschiedlichen Bildern bzw. Kombinationsbildern zusammengefasst und sowohl die Gesamthautfläche als auch die Fläche der identifizierten Hautregion bestimmt und zueinander in Verhältnis gesetzt. Die Berechnung erfolgt mittels der Auswerteeinrichtung der Recheneinrichtung.

In einem weiteren Verfahrensschritt wird der Grad der Rötung der Hautoberfläche, der Grad der Verschorfung bzw. Plaquebildung auf der Hautoberfläche und der Grad der Wölbung der Hautoberfläche in den identifizierten Hautregionen für jedes Segment mittels der Bildverarbeitungsvorrichtung bestimmt. Dazu kommen abermals an sich bekannte Bildverarbeitungsverfahren zum Einsatz. Beispielsweise wird zur Bestimmung des Grades der Rötung das Bild bzw. das Kombinationsbild in den LAB-Farbmodus übertragen und das Bild bzw. das Kombinationsbild dann derart gefiltert, dass stark rote Pixel sehr hell abgebildet werden und weniger rote Pixel entsprechend weniger hell abgebildet werden. Im Anschluss daran kann zum Beispiel auch noch eine Filterung der Helligkeitsverteilung mittels eines Gaus-Filters erfolgen. Dadurch kann eine gleichmäßigere Verteilung erreicht werden. Zur Berechnung der Wölbung bzw. des Grads der Wölbung können die Bilder bzw. Kombinationsbilder in Graustufenbilder überführt und entsprechend mit einem Sobel-Filter ersten Grades gefiltert werden. Dadurch entstehen in Bereichen mit großer Wölbung helle Bereiche. Der Grad der Verschorfung bzw. Plaquebildung kann beispielsweise durch eine Multiplikation des relativen Anteils an den identifizierten Hautregionen, auf denen Plaque bzw. Schorf identifiziert wurde, mit einem konstanten Faktor berechnet werden. Insgesamt werden als Ergebnis dieses Verfahrensschrittes für den Grad der Rötung, den Grad der Verschorfung und den Grad der Wölbung Zahlenwerte ermittelt, die beispielsweise ganzzahlig sind und zwischen 0 und 4 liegen.

In einem weiteren Verfahrensschritt erfolgt die Berechnung von je einem Segmentwert basierend auf dem Grad der Rötung der Hautoberfläche, dem Grad der Verschorfung bzw. Plaquebildung auf der Hautoberfläche, dem Grad der Wölbung der Hautoberfläche und dem Anteil der identifizierten Hautregionen an der Hautoberfläche pro Segment mittels der Auswerteeinrichtung. Die jeweiligen Segmentwerte werden so normiert, dass pro Segment mindestens ein Segmentwert von 0 (null) und höchstens ein Segmentwert von 6 erreicht wird.

Der Verfahrensschritt kann so ausgestaltet sein, dass die jeweilig ermittelten Grade der Rötung, Verschorfung und Wölbung addiert werden. Weiter kann eine Multiplikation des resultierenden Werts mit einem ganzzahligen Wert vorgesehen sein, der die anteilige Fläche der identifizierten Hautregion je Segment widerspiegelt. So kann zum Beispiel vorgesehen sein, dass der Wert zwischen 0 und 6 gemäß der nachfolgenden Tabelle an anteiliger Fläche der identifizierten Hautregion vergeben wird:

| 0: | 0 % |
|---|---|
| 1: | 1 - 9 % |
| 2: | 10 - 29 % |
| 3: | 30 - 49 % |
| 4: | 50 - 69 % |
| 5: | 70 - 89 % |
| 6: | 90 - 100 % |

Dabei entsprechen die Prozentangaben der anteiligen Fläche der identifizierten Hautregion.

In einem zusätzlichen Verfahrensschritt erfolgt die Berechnung eines gewichteten Gesamtwerts basierend auf den Segmentwerten und je einem Gewichtungsfaktor für jeden Segmentwert. Auch diese Berechnung wird mit der Auswerteeinrichtung der Recheneinrichtung durchgeführt. Beispielsweise kann vorgesehen sein, dass die Gewichtungsfaktoren zwischen 0,1 und 0,4 variieren. Der Gewichtungsfaktor kann beispielsweise dem durchschnittlichen Anteil der Hautoberfläche des jeweiligen Segments an der Gesamthautoberfläche eines durchschnittlichen Untersuchungskörpers entsprechen. Dann ist vorgesehen, dass die Gewichtungsfaktoren an sich in der Summe insgesamt einen Wert von 1 ergeben.

Des Weiteren kann vorgesehen sein, dass sich der Gesamtwert als Summe der gewichteten Segmentwerte ergibt.

In einem abschließenden Verfahrensschritt erfolgt die Ausgabe des Gesamtwerts und/oder der Segmentwerte mittels einer Ausgabeeinheit.

Durch das vorgeschlagene Verfahren wird es möglich die Erstellung des PASI bzw. des gewichteten Gesamtwerts von der Erfassung des Untersuchungskörpers, also von der Erzeugung der digitalen Bilder, bis zum endgültigen Berechnen und Ausgeben des PASI bzw. des Gesamtwerts oder der Segmentwerte, weitestgehend zu automatisieren und zudem hochgradig produzierbar zu machen.

Dadurch wird einerseits ermöglicht, dass auch Laien bzw. lediglich geringfügig geschultes Personal derartige Tätigkeiten bzw. die Erstellung des PASI bzw. des Gesamtwerts durchführen und/oder beaufsichtigen. Weiter kann durch die bereits vorhandene und stetig weitersteigende Rechenleistung bekannter Recheneinrichtungen das gesamte Verfahren nach Abschluss der Aufnahme der Bilder in äußerst kurzer Zeit durchgeführt werden. Vielmehr besteht sogar die Möglichkeit, dass sogar bereits während der Aufnahme der Bilder mit der Abarbeitung weiterer vorgesehener Verfahrensschritte begonnen wird, so dass die benötigte Zeit zur Durchführung des Verfahrens weiter verkürzt wird. Schließlich kann durch die Verlagerung der identifizierenden und berechnenden Verfahrensschritte auf eine Recheneinrichtung eine sehr hohe Reproduzierbarkeit der Ergebnisse erreicht werden.

Bei dem erfindungsgemäßen Verfahren erfolgt das Erstellen von Kombinationsbildern derart, dass identische Bereiche in zumindest zwei Bildern identifiziert werden und die Bilder im Anschluss an das Identifizieren identischer Bereiche derart zusammengefügt werden, dass die identifizierten Bereiche eine Überschneidung der ursprünglichen Bilder in den identifizierten Bereichen darstellt. Mit anderen Worten ausgedrückt bedeutet dies, dass im Rahmen des Erstellens der Kombinationsbilder Duplikate bzw. doppelt oder mehrfach abgebildete Bereiche des Untersuchungskörpers entfernt werden. Dadurch wird sichergestellt, dass einerseits der gesamte Untersuchungskörper abgebildet wurde, dass aber keine doppelte Abbildung des Untersuchungskörpers erfolgt ist, was zu einer Verfälschung des Gesamtwerts bzw. der Segmentwerte führen würde. Zudem sind die Kombinationsbilder besonders dann von Vorteil, wenn die vordefinierten Segmente so ausgebildet sind, dass sie sich auch bei einer gleichbleibenden Positionierung des Untersuchungskörpers relativ zum Abbildungssystem über mehrere Einzelbilder erstrecken würden. In einem derartigen Fall müssten die Segmente aufgeteilt und dementsprechend aufgeteilt identifiziert werden. Dies führt jedoch zu einer mit mehr Fehlern behafteten bzw. unsichereren Erkennung der Segmente.

Zudem ist es besonders wünschenswert, wenn im Rahmen des Verfahrens die Bilder und/oder die Kombinationsbilder auf der Ausgabeeinheit ausgegeben werden. Dies kann, wie sich weiter unten noch deutlicher zeigen wird, an unterschiedlichen Stellen bzw. bei unterschiedlichem Fortschritt des Gesamtverfahrens angezeigt und vorteilhaft sein. Grundsätzlich kann es jedoch vorteilhaft sein, wenn die Bilder und/oder Kombinationsbilder nach der Aufnahme und/oder Erzeugung auf der Ausgabeeinheit ausgegeben werden.

Soweit die Bilder und/oder Kombinationsbilder auf der Ausgabeeinheit ausgegeben werden, kann es besonders vorteilhaft sein, wenn die darin identifizierten Hautregionen bzw. Läsionen ebenfalls in den Bildern und/oder den Kombinationsbildern angezeigt werden. Dazu kann beispielsweise vorgesehen sein, dass farbliche Schattierungen bzw. farbliche halbtransparente Bereiche in den Bildern bzw. Kombinationsbildern angezeigt werden, die mit den identifizierten Hautregionen übereinstimmen.

Des Weiteren ist es besonders wünschenswert, wenn die identifizierten Hautregionen durch eine Eingabe an der Eingabeeinheit geändert und/oder entfernt und/oder zusätzliche identifizierte Hautregionen festgelegt werden können. Dadurch kann beispielsweise eine Korrektur vorgenommen werden, in der beispielsweise die automatische Identifizierung der Hautregionen, wie sie von der Bildverarbeitungsvorrichtung vorgenommen wird, aufgrund von Lichtverhältnissen oder dergleichen nicht vollständig oder korrekt erfolgt. In einem solchen Fall kann ein Bediener des Systems bzw. Anwender des Verfahrens bei der Anzeige der Bilder auf der Ausgabeeinheit und in einer Anzeige der identifizierten Hautregionen in den Bildern schnell und leicht die Regionen ausmachen, in denen die Identifizierung falsch, nicht vollständig oder gar nicht erfolgt ist und entsprechend mittels einer Eingabe über die Eingabeeinheit die Korrektur vornehmen. Damit wird ermöglicht, die Grenzen der automatischen Bildverarbeitung zu überwinden und trotzdem in den Genuss der Schnelligkeit der automatischen Bildverarbeitung zu kommen.

Außerdem kann es besonders vorteilhaft sein, wenn auch die Segmente auf der Ausgabeeinheit angezeigt werden. Dabei kann einerseits entweder eine schematisierte Ausgabe vordefinierter Bilder bzw. Kombinationsbilder erfolgen, in denen die Segmente angezeigt werden. Alternativ können die Segmente jedoch auch in den Bildern bzw. den Kombinationsbildern angezeigt werden. Auch dazu kann vorgesehen sein, dass die entsprechenden Pixel der Bilder bzw. Kombinationsbilder optisch, insbesondere farblich hervorgehoben werden.

Im letztgenannten Fall, in dem in den Bildern und/oder Kombinationsbildern, die auf einer Ausgabeeinheit angezeigt werden, auch die identifizierten Bereiche, die einem Segment zugeordnet sind, angezeigt werden, ist es weiter besonders vorteilhaft, wenn die Segmente durch eine Eingabe mittels der Eingabeeinheit geändert werden können. Auch diese Korrektur kann besonders vorteilhaft die Geschwindigkeit einer automatischen Identifizierung der Segmente in den Bildern bzw.

Kombinationsbildern mit einer nachträglichen manuellen und gegebenenfalls genaueren Korrektur verknüpfen.

Weiter ist es besonders wünschenswert, wenn die Bereiche Kopf, obere Extremitäten, Beine und Rumpf als Segmente in der identifizierten Hautoberfläche der Bilder bzw. Kombinationsbilder identifiziert werden. Dadurch wird eine Segmentierung erreicht, die auch bei einer manuellen Erstellung und Berechnung des PASI verwendet wird.

Um des Weiteren einer Fehlinterpretation durch die Bildverarbeitungsvorrichtung und/oder die Auswerteeinrichtung entgegenzuwirken, kann es vorteilhaft sein, wenn die Segmentwerte und/oder der gewichtete Gesamtwert durch eine Eingabe der Eingabeeinheit geändert werden können.

Weiter kann vorgesehen sein, dass der Übergang zwischen verschiedenen Verfahrensschritten des erfindungsgemäßen Verfahrens als Reaktion auf eine Eingabe der Eingabeeinheit ausgelöst wird. Dies vermindert zwar geringfügig die Gesamtgeschwindigkeit zum Erreichen des gewichteten Gesamtwerts bzw. zum Erreichen des PASI, es wird jedoch gleichzeitig ermöglicht, die einzelnen Verfahrensschritte zu überprüfen und gegebenenfalls, wie oben bereits beschrieben, auf die einzelnen Verfahrensschritte mit einer Eingabe korrigierend oder zumindest verifizierend einzuwirken.

Um dem Verfahren über einen einzelnen Vorgang betreffend einen Untersuchungskörper hinaus weiter besondere Vorteile zu verleihen, ist es besonders wünschenswert, wenn der berechnete gewichtete Gesamtwert in der Speichereinrichtung gespeichert wird. Dadurch ist es möglich, die Fortschritte einer Therapie bzw. die Entwicklung des Krankheitsbildes zu dokumentieren und zu vergleichen. Neben der Speicherung des gewichteten Gesamtwerts kann es auch vorteilhaft vorgesehen sein, dass die Segmentwerte, die berechnete anteilige Fläche der identifizierten Hautregionen je Segment, die identifizierten Hautregionen, die identifizierten vordefinierten Segmente und die identifizierte Hautoberfläche des Untersuchungskörpers zumindest mittelbar im Anschluss an ihre Identifizierung in der Speichereinrichtung gespeichert werden. Dadurch kann neben einem Vergleich der Ergebnisse, also der Segmentwerte oder des gewichteten Gesamtwerts auch eine Veränderung hinsichtlich der oben genannten Merkmale im Verlauf einer wiederholten Anwendung des Verfahrens auf dem gleichen Untersuchungskörper festgestellt werden.

Die erfindungsgemäße Aufgabe wird ebenfalls durch ein Ganzkörperbildaufnahme- und Bildverarbeitungssystem gelöst, welches ein Abbildungssystem zur Aufnahme digitaler Bilder eines Untersuchungskörpers anhand einer Mehrzahl vorgegebener Positionierungen des Untersuchungskörpers umfasst, wobei das Abbildungssystem zumindest eine Aufnahmeeinrichtung und eine automatische Verstelleinrichtung aufweist. Weiter ist eine mit dem Abbildungssystem verbundene Speichereinrichtung, die zumindest zur Speicherung von digitalen Bildern ausgestaltet ist, sowie eine Recheneinrichtung umfassend eine Bildverarbeitungsvorrichtung und eine Auswerteeinrichtung, wobei die Recheneinrichtung mit der Speichereinrichtung verbunden ist und zudem eine Ausgabeeinheit und eine Eingabeeinheit vom Ganzkörperbildaufnahme- und Bildverarbeitungssystem umfasst, vorgesehen.

Der erfindungsgemäße Charakter des Systems wird dadurch erreicht, dass die Bildverarbeitungseinrichtung dazu eingerichtet ist, Kombinationsbilder aus zumindest zwei unterschiedlichen Bildern der gleichen Positionierung des Untersuchungskörpers zu erzeugen, wobei das Erstellen von Kombinationsbildern das Identifizieren von identischen Bereichen in zumindest zwei Bildern und das Zusammenfügen der Bilder mit einer Überschneidung in den identifizierten Bereichen umfasst, Hautoberfläche des Untersuchungskörpers in den Bildern bzw. Kombinationsbildern zu identifizieren, vordefinierte Segmente der identifizierten Hautoberfläche in den Bildern bzw. Kombinationsbildern zu identifizieren, Hautregionen in den Bildern und/oder Kombinationsbildern, welche eine Rötung oder Schorfbildung bzw. Plaquebildung aufweisen, zu identifizieren sowie einen Grad der Rötung der Hautoberfläche, einen Grad der Verschorfung der Hautoberfläche oder einen Grad der Wölbung der Hautoberfläche in den identifizierten Hautregionen für jedes Segment zu bestimmen, und die Rechenrichtung dazu eingerichtet ist, einen Anteil an identifizierten Hautregionen an der identifizierten Hautoberfläche je Segment, Segmentwerte basierend auf dem Grad der Rötung der Hautoberfläche, dem Grad der Verschorfung der Hautoberfläche, dem Grad der Wölbung der Hautoberfläche und dem Anteil der Hautoberfläche an identifizierten Hautregionen zu berechnen und einen gewichteten Gesamtwert basierend auf den Segmentwerten und je einem Gewichtungsfaktor für jeden Segmentwert zu berechnen.

Durch das erfindungsgemäße Ganzkörperbildaufnahme- und Bildverarbeitungssystem wird ermöglicht, den gewichteten Gesamtwert in Form eines PASI schnell, zuverlässig, reproduzierbar und ohne Beteiligung hochgradig spezialisierter Bediener bzw. Benutzer zu ermitteln. Zudem wird durch die Ausgestaltung des Systems ermöglicht, den Fortlauf einer Therapie bzw. einer Erkrankung zu überwachen und zu vergleichen.

Gemäß einer vorteilhaften Ausgestaltung ist die Speichereinrichtung auch zur Speicherung von Daten betreffend den Untersuchungskörper von Kombinationsbildern, von Segmentwerten und gewichteten Gesamtwerten eingerichtet. Dadurch kann einerseits die automatische Ansteuerung des Abbildungssystems und damit die automatische Aufnahme digitaler Bilder eines Untersuchungskörpers erreicht werden. Die Speicherung von Kombinationsbildern, Segmentwerten und gewichteten Gesamtwerten erlaubt zudem die Abgleichung von vorangehenden Ergebnissen der Benutzung des Systems für den identischen Untersuchungskörper. Zudem kann auch vorgesehen sein, dass die Speichereinrichtung zur Speicherung der mittels der Bildverarbeitungsvorrichtung erzeugten Daten betreffend die Bilder bzw. Kombinationsbilder eingerichtet ist. Unter anderem können beispielsweise die identifizierten Hautregionen, der identifizierte Anteil der Hautregionen an der Gesamthautoberfläche oder andere mittels der Bildverarbeitungsvorrichtung aus den Bildern gewonnene Daten gespeichert werden. Auch dies ermöglicht eine verbesserte Analyse des zeitlichen Verlaufs der Erkrankung bzw. der Behandlung der Erkrankung.

Ebenfalls vorteilhaft ist, dass das Ganzkörperbildaufnahme- und Bildverarbeitungssystem so ausgestaltet ist, dass die Verstelleinrichtung durch eine Steuereinrichtung gesteuert wird und die Aufnahmeeinrichtung in Abhängigkeit von Daten betreffend den Untersuchungskörper und der Positionierung des Untersuchungskörpers steuert. Beispielsweise kann vorgesehen sein, dass mit der Eingabeeinheit Daten betreffend den Untersuchungskörper, wie beispielsweise Größe und Geschlecht, eingegeben und in der Speichereinrichtung hinterlegt werden. Die Steuereinrichtung kann dann so ausgestaltet sein, dass sie auf diese Daten betreffend den Untersuchungskörper zugreifen kann und entsprechende Steuersignale zur Ansteuerung der Aufnahmeeinrichtung erzeugen kann. Dadurch werden einerseits eine genaue sowie eine schnelle Aufnahme der digitalen Bilder des Untersuchungskörpers ermöglicht. Zudem kann damit auch eine reproduzierbare Aufnahme von Bildern bei einer wiederholten Benutzung bzw. Verwendung des Systems erreicht werden.

Weiter vorteilhaft ist es, wenn die Ausgabeeinheit als Bildschirm und/oder als Drucker ausgestaltet ist. Besonders vorteilhaft ist es dabei, wenn das System sowohl einen Bildschirm als auch einen Drucker umfasst. Mittels des Bildschirms kann beispielsweise eine Benutzerinteraktion stattfinden und mittels des Druckers können beispielsweise Ergebnisse, die mit dem System erzeugt werden, festgehalten und dokumentiert werden.

Im Folgenden werden beispielhafte Ausgestaltungen der vorliegenden Erfindung anhand lediglich schematischer Zeichnungen beispielhaft erläutert.

Es zeigen:

Fig. 1 ein schematisiertes Ablaufdiagramm eines Teils des erfindungsgemäßen Verfahrens;

Fig. 2 eine schematisierte Ablaufdarstellung eines Teils des erfindungsgemäßen Verfahrens gemäß einer Abwandlung;

Fig. 3 eine schematisierte Abbildung des erfindungsgemäßen Systems; und

Fig. 4 eine schematisierte Darstellung von Bildern, Kombinationsbildern und entsprechenden Positionierungshilfen zur Positionierung des Untersu-chungskörpers.

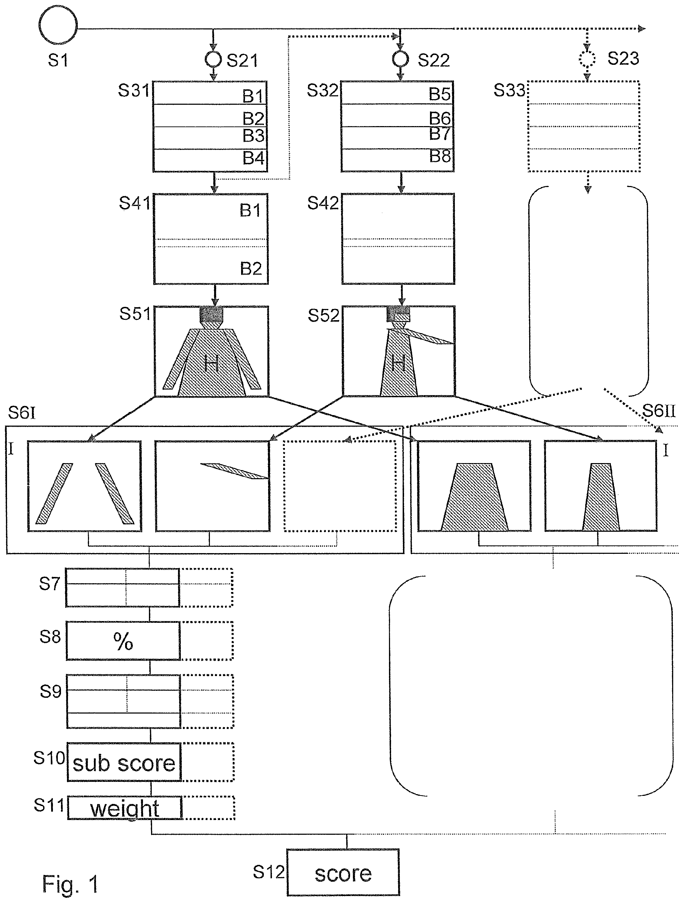

Fig. 1 zeigt einen Ausschnitt aus dem erfindungsgemäßen Verfahren, welches mit dem Verfahrensschritt S1 beginnt und mit dem Verfahrensschritt S12 endet. Im Verfahrensschritt S1 wird über eine Eingabeeinheit ein Datensatz bzw. Daten betreffend den Untersuchungskörper eingegeben oder ein entsprechender Datensatz aus der Speichereinrichtung mittels der Eingabeeinheit ausgewählt.

In den Schritten S21, S22 und S23 sowie in den weiteren nicht in Fig. 1 dargestellten Verfahrensschritten erzeugt eine Steuereinrichtung des Abbildungssystems daraus Steuerbefehle zur Steuerung der Verstelleinrichtung des Abbilddungssystems, mit dem die Aufnahmeeinrichtung angesteuert wird. Die in den Schritten S21, S22 und S23 erzeugten Signale betreffen dabei jeweils eine unterschiedliche Positionierung des Untersuchungskörpers bezüglich des Abbildungssystems.

In den Verfahrensschritten S31 bis S33 und den weiteren vorgesehenen aber in Fig. 1 nicht dargestellten Verfahrensschritten erfolgt die Aufnahme der Bilder mittels des Abbildungssystems. Beispielsweise werden im Schritt S31 in einer ersten Positionierung des Untersuchungskörpers die Bilder B1 bis B4 erzeugt. Entsprechend werden im Schritt S32 die Bilder B5 bis B8 erzeugt.

In den Verfahrensschritten S41 und S42 werden, wenn es für die jeweilige Positionierung des Untersuchungskörpers vorgesehen ist, aus allen im Schritt S31 oder zumindest aus zwei im Schritt S31 aufgenommenen Bildern Kombinationsbilder erzeugt. Dabei kann beispielsweise vorgesehen sein, dass der Schritt S41 und der Schritt S22 gleichzeitig eingeleitet werden, was durch die strichlinierte Verbindung in Fig. 1 angedeutet ist. Mit anderen Worten ausgedrückt bedeutet dies, dass während die Bildverarbeitungsvorrichtung die Bilder des Schritts S31 zumindest teilweise zu Kombinationsbildern kombiniert, bereits neue Steuersignale erzeugt bzw. ausgeführt werden können, um die im Schritt S32 aufzunehmenden Bilder B5 bis B8 aufzunehmen. Gleiches gilt auch für die parallele Durchführung anderer Verfahrensschritte. Die in Fig. 1 dargestellten Abläufe betreffen eine mögliche Abfolge der einzelnen Schritte. Eine abweichende Abfolge ist jedoch ebenfalls denkbar, soweit die Reihenfolge derart unverändert bleibt, dass die jeweils in einem Verfahrensschritt benötigten Zwischenergebnisse oder Vorverarbeitungsschritte vor dem jeweiligen Verfahrensschritt erzeugt werden.

Der Schritt S42 entspricht dementsprechend auch einer Erzeugung von Kombinationsbildern aus den Bildern des Schritts S32. In den womöglich zeitversetzt ausgeführten Schritten S51 und S52 wird aus den aufgenommenen Bildern bzw. erzeugten Kombinationsbildern mittels der Bildbearbeitungsvorrichtung die in den Bildern bzw. Kombinationsbildern abgebildete Hautoberfläche H extrahiert bzw. diese identifiziert. Wie in den Schritten S51 und S52 angedeutet, handelt es sich bei den beispielhaften Kombinationsbildern um eine Rückansicht bzw. eine Seitenansicht des Untersuchungskörpers.

Entsprechend kann in den durch den Verfahrensschritt S23 eingeleiteten Zweig des Verfahrens eine weitere Positionierung des Untersuchungskörpers bearbeitet werden. Gleiches gilt für weitere nicht dargestellte Verfahrenszweige.

In den Schritten S6I und S6II wird entsprechend aus den vorangehend identifizierten Hautoberflächen die jeweils einem Segment zugeordnete Oberfläche extrahiert. Beispielsweise werden im Verfahrensschritt S6I die Pixel der Bilder bzw. Kombinationsbilder extrahiert oder identifiziert, die das Segment der oberen Extremitäten abbilden bzw. darstellen. Entsprechend wird im Verfahrensschritt S6II die Hautoberfläche bzw. die entsprechenden Pixel der jeweiligen Bilder oder Kombinationsbilder, die dem Segment Rumpf zugeordnet sind, extrahiert und zusammengefasst.

Die weiteren Verfahrensschritte S7 bis S12 werden lediglich für das im Verfahrensschritt S6I zusammengefasste Segment der oberen Extremitäten beschrieben. Die Verfahrensschritte S7 bis S12 finden für die verbleibenden Segmente, wie beispielsweise im Verfahrensschritt S6II identifiziert, eine analoge Anwendung.

Im Verfahrensschritt S7 wird in den dem Segment zugeordneten identifizierten Hautoberflächen eine Identifizierung von Hautregionen vorgenommen, die eine Rötung aufweisen oder eine Schorf- bzw. Plaquebildung aufweisen.

Im anschließenden Verfahrensschritt S8 wird der Anteil der im Schritt S7 bestimmten Hautregion an der gesamten dem Segment zugeordneten Hautoberfläche bestimmt. Dabei erfolgt eine Umwandlung in einen ganzzahligen Zahlenwert zwischen 0 und 6 gemäß der obigen Tabelle.

Im Schritt S9 wird mittels entsprechender Algorithmen der Grad der Rötung der Hautoberfläche, der Grad der Verschorfung der Oberfläche sowie der Grad der Wölbung der Hautoberfläche in den identifizierten Hautregionen berechnet. Die Berechnung umfasst dabei eine Normierung auf einen ganzzahligen Zahlenwert zwischen 0 und 4.

Aus den Ergebnissen der Verfahrensschritte S8 und S9 wird im anschließenden Verfahrensschritt S10 ein Segmentwert (Subscore) berechnet. Im Anschluss daran wird im Verfahrensschritt S11 der Segmentwert mit einem Gewichtungsfaktor gewichtet. Aus dem in Schritt S11, wie in den vergleichbaren Schritten anderer Segmente gewonnenen Segmentwert, wird im abschließenden Verfahrensschritt S12 der Gesamtwert bzw. der gewichtete Gesamtwert errechnet und dieser auf einer Ausgabeeinheit ausgegeben.

Insgesamt kann die auf die entsprechenden Bildverarbeitungsschritte aufbauende rein rechnerische Bestimmung des PASI-Gesamtwerts bzw. der Score wie folgt zusammengefasst werden:

$$Score = \sum_{i=1}^{n} g_i \cdot r_i \cdot (GR_i + GS_i + GW_i),$$

mit:

i = i-tes Segment;
n = Anzahl der Segmente = 4;
$GR_i$ = Grad der Rötung, {0, 1, ..., 4};
$GS_i$ = Grad der Schorfbildung, {0, 1, ..., 4};
$GW_i$ = Grad der Wölbung, {0, 1, ..., 4}
r = anteilige Fläche der identifizierten Hautoberfläche, {0, 1, ..., 6};
$g_i$ = Gewichtungsfaktor, {0,1; 0,2; 0,3; 0,4}.

Bei einer Berechnung mit vier Segmenten können zum Beispiel Segmente für den Kopf- und Nackenbereich, die oberen Extremitäten, den Rumpf und die unteren Extremitäten vorgesehen sein. In diesem Fall kann vorgesehen sein, dass dem Segment des Kopf- und Nackenbereichs ein Gewichtungsfaktor von $g_1=0,1$, dem Segment der oberen Extremitäten ein Gewichtungsfaktor von $g_2=0,2$, dem Segment des Rumpfs ein Gewichtungsfaktor von $g_3=0,3$ und dem Segment der unteren Extremitäten ein Gewichtungsfaktor von $g_4=0,4$ zugeordnet wird. Damit ergibt die Gesamtsumme der Gewichtungsfaktoren gerade 1.

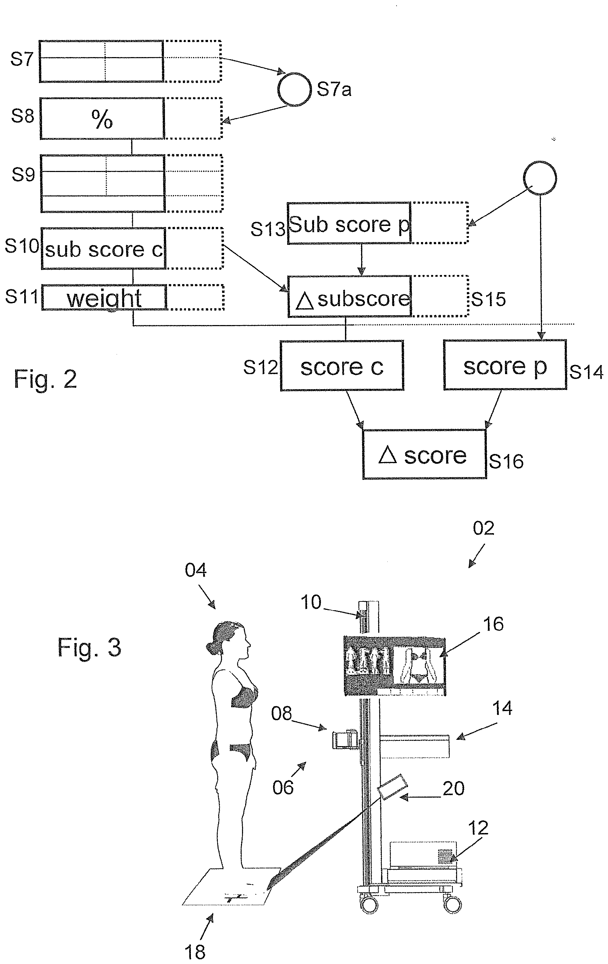

Fig. 2 zeigt eine Abwandlung des erfindungsgemäßen Verfahrens. In Fig. 2 ist vorgesehen, dass im Anschluss an den Verfahrensschritt S7, also im Anschluss an die Identifizierung der Hautregionen, die eine Rötung oder Schorfbildung aufweisen, eine Eingabe mittels der Eingabeeinheit im Schritt S7a erfolgt. Dabei kann einerseits vorgesehen sein, dass die Eingabe im Schritt S7a lediglich zur Überleitung zwischen Schritt S7 und Schritt S8 bzw. zur Initiierung des Schritts S8 dient. Andererseits kann jedoch auch vorgesehen sein, dass mittels der Eingabe an der Eingabeeinheit die Ergebnisse des Schritts S7 korrigiert werden, bevor der Schritt S8 initiiert wird.

Des Weiteren stellt die Abwandlung der Fig. 2 eine Durchführung des Verfahrens für einen Untersuchungskörper dar, für den in der Vergangenheit bereits das Verfahren durchgeführt wurde, wobei die Ergebnisse oder Zwischenergebnisse des Verfahrens in der Speichereinrichtung des Systems hinterlegt wurden. Entsprechend wird in den Verfahrensschritten S13 und S14 ein Segmentwert und ein gewichteter Gesamtwert aus der Speichereinrichtung entnommen und mit dem im Schritt S10 berechneten Segmentwert bzw. mit dem im Schritt S12 berechneten gewichteten Gesamtwert verglichen. Dadurch wird in den Verfahrensschritten S15 und S16 jeweils die Änderung des Segmentwerts bzw. die Änderung des gewichteten Gesamtwerts berechnet und gegebenenfalls auf der Ausgabeeinheit des Systems ausgegeben.

Fig. 3 zeigt ein erfindungsgemäßes Ganzkörperbildaufnahme- und Bildverarbeitungssystem 02 sowie einen Untersuchungskörper 04. Der Untersuchungskörper 04 wird mittels eines Abbildungssystems 06 in Form digitaler Bilder abgebildet, wobei das Abbildungssystem 08 eine Aufnahmeeinrichtung 06 sowie eine Verstelleinrichtung 10 aufweist. Mittels der Verstelleinrichtung 10 kann die Aufnahmeeinrichtung 06 in der Höhe verstellt werden. Die Verstelleinrichtung kann dabei durch eine nicht abgebildete Steuereinrichtung gesteuert werden, die auch die Auslösung der Aufnahmeeinrichtung steuern kann. Die zur Ansteuerung der Verstelleinrichtung notwendigen Daten betreffend den Untersuchungskörper können beispielsweise in einer Speichereinrichtung hinterlegt sein, welche in der Recheneinrichtung 12 angeordnet ist. Alternativ können die Daten betreffend den Untersuchungskörper auch mittels einer Eingabeeinheit 14 eingegeben werden.

Das erfindungsgemäße System weist weiter eine Ausgabeeinheit 16 in Form eines Bildschirms auf. Zur Positionierung des Untersuchungskörpers bezüglich des Abbildungssystems ist in Fig. 3 eine mögliche Ausgestaltung dargestellt, in der der Untersuchungskörper auf einer Fußmatte 18 positioniert wird, wobei die Fußmatte 18 entsprechende graphische Darstellungen, beispielsweise in Form von Piktogrammen enthält, die die jeweilige Positionierung des Untersuchungskörpers vermitteln.

Um den Abstand zwischen Untersuchungskörper und dem Abbildungssystem zu bestimmen, kann zudem eine Messeinrichtung 20 vorgesehen sein, die mittels geeigneter Verfahren, wie beispielsweise optischer Messverfahren, den Abstand zwischen dem Ganzkörperbildaufnahme- und Bildverarbeitungssystem und der Fußmatte 18 und damit mittelbar den Abstand zum Untersuchungskörper bestimmt.

Die erfindungsgemäße Vorrichtung weist dabei in der Recheneinrichtung 12 eine Bildverarbeitungsvorrichtung und eine Auswerteeinrichtung auf, die zur Durchführung des erfindungsgemäßen Verfahrens in Verbindung mit der Speichereinrichtung und dem Abbildungssystem eingerichtet ist.

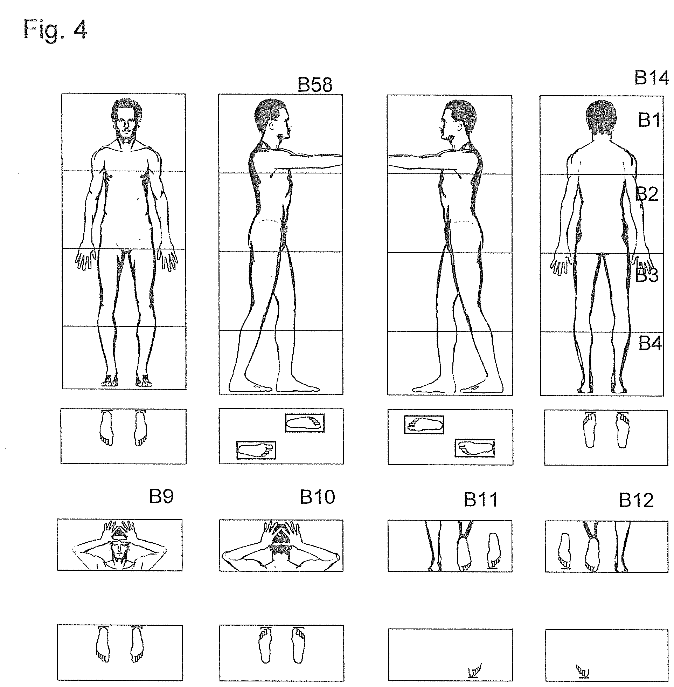

Fig. 4 zeigt insgesamt vier Kombinationsbilder sowie vier Bilder, die aus den digitalen Aufnahmen des Abbildungssystems hervorgehen bzw. mittels dieser generiert werden. Zudem zeigt die Fig. 4 in den jeweiligen Kombinationsbildern bzw. Bildern die auf der Fußmatte 18 vorgesehenen Piktogramme, die dazu dienen, die jeweilige Positionierung des Untersuchungskörpers bezüglich des Abbildungssystems einzunehmen. Die Fig. 4 zeigt weiter das in Fig.1 bereits beispielhaft dargestellte Kombinationsbild aus den Bilder B1 bis B4. Gleichermaßen zeigt die Fig. 4 auch das in Fig. 1 bereits schematisch dargestellte Kombinationsbild B58 aus den Bildern B5 bis B8. Auch die einzelnen Bilder B9 bis B12, wie sie zur Durchführung des erfindungsgemäßen Verfahrens von dem Abbildungssystem aufgenommen werden, sind in Fig. 4 dargestellt.

## Patentansprüche

**1.** Verfahren zum Betrieb eines Ganzkörperbildaufnahme- und Bildverarbeitungssystems (02) aufweisend ein automatisches Abbildungssystem (08), eine Speichereinrichtung sowie eine Recheneinrichtung (12) umfassend eine Bildverarbeitungsvorrichtung, eine Auswerteeinrichtung sowie eine Ausgabeeinheit (16) und eine Eingabeeinheit (14) zumindest umfassend die Verfahrensschritte:

- Aufnahme digitaler Bilder (B1 - B12) eines Untersuchungskörpers (04) anhand einer Mehrzahl vorgegebener Positionierungen des Untersuchungskörpers (04) durch das Abbildungssystem (S31, S32);
- Erstellen von zumindest einem Kombinationsbild (B14, B58) aus zumindest zwei unterschiedlichen Bildern (B1 - B4, B5 - B8) der gleichen Positionierung des Untersuchungskörpers mittels der Bildverarbeitungsvorrichtung (S41, S42);
- Identifizierung von Hautoberfläche (H) des Untersuchungskörpers in den Bildern und/oder Kombinationsbildern mittels der Bildbearbeitungsvorrichtung (S51, S52);
- Identifizieren vordefinierter Segmente der identifizierten Hautoberfläche (H) in den Bildern und/oder Kombinationsbildern mittels der Bildverarbeitungsvorrichtung (S6I, S6II);
- Identifizieren von Hautregionen in den Bildern und/oder Kombinationsbildern, die Rötungen oder Schorfbildung aufweisen mittels der Bildverarbeitungsvorrichtung (S7);
- Berechnen der anteiligen Fläche der identifizierten Hautregion je Segment mittels der Auswerteeinrichtung (S8);
- Bestimmen eines Grades der Rötung der Hautoberfläche, des Grades der Verschorfung der Hautoberfläche und eines Grades der Wölbung der Hautoberfläche in den identifizierten Hautregionen pro Segment mittels der Bildverarbeitungsvorrichtung (S9);
- Berechnen von je einem Segmentwert basierend auf dem Grad der Rötung der Hautoberfläche, dem Grad der Verschorfung der Hautoberfläche, dem Grad der Wölbung der Hautoberfläche und dem Anteil der der identifizierten Hautregionen der Hautoberfläche pro Segment mittels der Auswerteeinrichtung (S10);
- Berechnen eines gewichteten Gesamtwerts basierend auf den Segmentwerten und je einem Gewichtungsfaktor für jeden Segmentwert mittels der Auswerteeinrichtung (S11);
- Ausgabe des Gesamtwerts und/oder der Segmentwerte mittels einer Ausgabeeinheit (16) (S12),

**dadurch gekennzeichnet,**

**dass** das Erstellen von Kombinationsbildern das Identifizieren von identischen Bereichen in zumindest zwei Bildern und das Zusammenfügen der Bilder mit einer Überschneidung in den

**2.** Verfahren nach Anspruch 1,
**dadurch gekennzeichnet,**
**dass** die Bilder und/oder die Kombinationsbilder auf der Ausgabeeinheit (16) ausgegeben werden.

**3.** Verfahren nach Anspruch 2,
**dadurch gekennzeichnet,**
**dass** die identifizierten Hautregionen in den Bildern und/oder Kombinationsbildern angezeigt werden.

**4.** Verfahren nach Anspruch 3,
**dadurch gekennzeichnet,**
**dass** die identifizierten Hautregionen durch eine Eingabe an der Eingabeeinheit (14) geändert und/oder entfernt und/oder zusätzliche identifizierte Hautregionen festgelegt werden können.

**5.** Verfahren nach einem der Ansprüche 2 bis 4,
**dadurch gekennzeichnet,**
**dass** die Segmente insbesondere in den Bildern und/oder Kombinationsbildern angezeigt werden.

**6.** Verfahren nach Anspruch 5,
**dadurch gekennzeichnet,**
**dass** die Segmente durch eine Eingabe der Eingabeeinheit (14) geändert werden können.

**7.** Verfahren nach einem der Ansprüche 1 bis 6,
**dadurch gekennzeichnet,**
**dass** die Bereiche Kopf, obere Extremitäten, Beine und Rumpf als Segmente in der identifizierten Hautoberfläche in den Bildern und/oder Kombinationsbildern identifiziert werden.

**8.** Verfahren nach einem der Ansprüche 1 bis 7,
**dadurch gekennzeichnet,**
**dass** die Segmentwerte und/oder der gewichtete Gesamtwert durch eine Eingabe der Eingabeeinheit (14) geändert werden können.

**9.** Verfahren nach einem der Ansprüche 1 bis 8,
**dadurch gekennzeichnet,**
**dass** der Übergang zwischen verschiedenen Verfahrensschritten als Reaktion auf eine Eingabe der Eingabeeinheit erfolgt.

**10.** Verfahren nach einem der Ansprüche 1 bis 9,
**dadurch gekennzeichnet,**
**dass** der gewichtete Gesamtwert in der Speichereinrichtung gespeichert wird.

**11.** Ganzkörperbildaufnahme- und Bildverarbeitungssystem aufweisend ein Abbildungssystem (08) zur Aufnahme digitaler Bilder eines Untersuchungskörpers (04) anhand einer Mehrzahl vorgegebener Positionierungen des Untersuchungskörpers (04), wobei das Abbildungssystem (08) zumindest eine Aufnahmeeinrichtung (06) und eine automatische Verstelleinrichtung (10) aufweist, eine mit dem Abbildungssystem (08) verbundene Speichereinrichtung, die zumindest zur Speicherung von digitalen Bildern ausgestaltet ist, sowie eine Recheneinrichtung (12) umfassend eine Bildverarbeitungsvorrichtung, eine Auswerteeinrichtung, eine Ausgabeeinheit (16) und eine Eingabeeinheit (14), wobei die Recheneinrichtung mit der Speichereinrichtung verbunden ist, wobei die Bildverarbeitungsvorrichtung dazu eingerichtet ist, Kombinationsbilder aus zumindest zwei unterschiedlichen Bildern der gleichen Positionierung des Untersuchungskörpers zu erzeugen, wobei das Erstellen von Kombinationsbildern das Identifizieren von identischen Bereichen in zumindest zwei Bildern und das Zusammenfügen der Bilder mit einer Überschneidung in den identifizierten Bereichen umfasst, Hautoberfläche des Untersuchungskörpers in den Bildern und/oder Kombinationsbildern zu identifizieren, vordefinierte Segmente der identifizierten Hautoberfläche in den Bildern und/oder Kombinationsbildern zu identifizieren, Hautregionen in den Bildern und/oder Kombinationsbildern, die Rötungen oder Schorfbildung aufweisen, zu identifizieren sowie einen Grad der Rötung der Hautoberfläche, einen Grad der Verschorfung der Hautoberfläche und einen Grad der Wölbung der Hautoberfläche in den identifizierten Hautregionen pro Segment zu bestimmen,
und die Auswerteeinrichtung dazu eingerichtet ist, die anteilige Fläche der identifizierten Hautregionen je Segment zu berechnen, je einen Segmentwert basierend auf dem Grad der Rötung der Hautoberfläche, dem Grad der Verschorfung der Hautoberfläche, dem Grad der Wölbung der Hautoberfläche und dem Anteil der Hautoberfläche der identifizierten Hautregionen zu berechnen und einen gewichteten Gesamtwert basierend auf den Segmentwerten und je einem Gewichtungsfaktor für jeden Segmentwert zu berechnen.

**12.** Ganzkörperbildaufnahme- und Bildverarbeitungssystem nach Anspruch 11,
**dadurch gekennzeichnet,**
**dass** die Speichereinrichtung zur Speicherung von Daten betreffend den Untersuchungskörper von Kombinationsbildern, von Segmentwerten und gewichteten Gesamtwerten eingerichtet ist.

**13.** Ganzkörperbildaufnahme- und Bildverarbeitungssystem nach Anspruch 11 oder 12,
**dadurch gekennzeichnet,**
**dass** die Verstelleinrichtung (10) über eine Steuereinrichtung gesteuert wird, welche die Aufnahmeeinrichtung in Abhängigkeit von Daten betreffend den Untersuchungskörper (04) und der Positionierung des Untersuchungskörpers (04) steuert.

**14.** Ganzkörperbildaufnahme- und Bildverarbeitungssystem nach einem der Ansprüche 11 bis 13, **dadurch gekennzeichnet, dass** die Ausgabeeinheit (16) als Bildschirm und/oder als Drucker ausgestaltet ist.

**Claims**

**1.** A method for operating a full-body image capturing and image processing system (02) having an automatic imaging system (08), a memory and a computing means (12) comprising an image processing device, an evaluating means and an output unit (16) and an input unit (14), comprising at least the method steps of:

- capturing digital images (B1 - B12) of a body to be examined (04) by means of the imaging system using a plurality of predetermined positions of the body to be examined (04) (S31, S32);
- creating at least one combined image (B14, B58) from at least two different images (B1 - B4, B5 - B8) of the same position of the body to be examined by means of the image processing device (S41, S42);
- identifying skin surface (H) of the body to be examined in the images and/or combined images by means of the image processing device (S51, S52);
- identifying predefined segments of the identified skin surface (H) in the images and/or combined images by means of the image processing device (S6I, S6II);
- identifying skin regions that show reddening or scabbing in the images and/or combined images by means of the image processing device (S7);
- calculating the proportional area of the identified skin regions per segment by means of the evaluating means (S8);
- determining a degree of reddening of the skin surface, the degree of scabbing of the skin surface and a degree of curvature of the skin surface in the identified skin regions per segment by means of the image processing device (S9);
- calculating one segment value based on each of the degree of reddening of the skin surface, the degree of scabbing of the skin surface, the degree of curvature of the skin surface and the proportion of the identified skin regions of the skin surface per segment by means of the evaluating means (S10);
- calculating a weighted total value based on the segment values and one weighting factor for each segment value by means of the evaluating means (S11);
- outputting the total value and/or the segment values by means of an output unit (16) (S12),

**characterised in that** creating combined images comprises identifying identical areas in at least two images and merging the images with an overlap in the identified areas.

**2.** The method according to claim 1, **characterised in that** the images and/or the combined images are outputted on the output unit (16).

**3.** The method according to claim 2, **characterised in that** the identified skin regions are displayed in the images and/or combined images.

**4.** The method according to claim 3, **characterised in that** the identified skin regions can be changed and/or removed and/or additional identified skin regions can be defined by an input at the input unit (14).

**5.** The method according to any one of claims 2 to 4, **characterised in that** the segments are displayed in particular in the images and/or combined images.

**6.** The method according to claim 5, **characterised in that** the segments can be changed by an input of the input unit (14).

**7.** The method according to any one of claims 1 to 6, **characterised in that** the areas head, upper extremities, legs and torso are identified in the images and/or combined images as segments in the identified skin surface.

**8.** The method according to any one of claims 1 to 7, **characterised in that** the segment values and/or the weighted total value can be changed by an input of the input unit (14).

**9.** The method according to any one of claims 1 to 8, **characterised in that** the transition between different method steps happens in response to an input of the input unit.

**10.** The method according to any one of claims 1 to 9, **characterised in that** the weighted total value is stored in the memory.

**11.** A full-body image capturing and image processing system having an imaging system (08) for capturing digital images of a body to be examined (04) using a plurality of predetermined positions of the body to be examined (04), the imaging system (08) having at least a capturing means (06) and an automatic adjusting means (10), a memory connected to the imaging system (08) and at least configured to store digital images, and a computing means (12) comprising an image processing device, an evaluating means, an output unit (16) and an input unit (14), the computing means being connected to the memory, wherein the image processing device is configured to create combined images from at least two different images of the same position of the body to be examined, said creation of combined images comprising the identification of identical areas in at least two images and the merging of the images with an overlap in the identified areas, the image processing device is configured to identify skin surface of the body to be examined in the images and/or combined images, to identify predefined segments of the identified skin surface in the images and/or combined images, to identify skin regions that show reddening or scabbing in the images and/or combined images, and to determine a degree of reddening of the skin surface, a degree of scabbing of the skin surface and a degree of curvature of the skin surface in the identified skin regions per segment, and wherein the evaluating means is configured to calculate the proportional area of the identified skin regions per segment, to calculate one segment value based on each of the degree of reddening of the skin surface, the degree of scabbing of the skin surface, the degree of curvature of the skin surface and the proportion of the skin surface of the identified skin regions and to calculate a weighted total value based on the segment values and one weighting factor for each segment value.

12. The full-body image capturing and image processing system according to claim 11,
**characterised in that**
the memory is configured to store data regarding the body to be examined, combined images, segment values and weighted total values.

13. The full-body image capturing and image processing system according to claim 11 or 12,
**characterised in that**
the adjusting means (10) is controlled via a controller, which controls the capturing means as a function of data regarding the body to be examined (04) and of the position of the body to be examined (04).

14. The full-body image capturing and image processing system according to any one of claims 11 to 13,
**characterised in that**
the output unit (16) is realised as a screen and/or as a printer.

**Revendications**

1. Procédé d'opération d'un système (02) de prise de vues du corps entier et de traitement d'images ayant un système d'imagerie (08) automatique, un support de stockage ainsi qu'un moyen de calcul (12) comprenant un dispositif de traitement d'images, un moyen d'évaluation ainsi qu'une unité de sortie (16) et une unité d'entrée (14),
comprenant au moins les étapes de procédé suivantes :

- capturer des images numériques (B1 - B12) d'un corps à examiner (04) à l'aide d'une pluralité de positions prédéterminées du corps á examiner (04) moyennant le système d'imagerie (S31, S32) ;
- générer au moins une image combinée (B14, B58) à partir d'au moins deux images différentes (B1 - B4, B5 - B8) de la même position du corps à examiner à travers le dispositif de traitement d'images (S41, S42) ;
- identifier de la surface cutanée (H) du corps à examiner dans les images et/ou dans les images combinées à travers le dispositif de traitement d'images (S51, S52) ;
- identifier des segments prédéfinis de la surface cutanée (H) identifiée dans les images et/ou dans les images combinées à travers le dispositif de traitement d'images (S6I, S6II) ;
- identifier des zones cutanées qui présentent des rougeurs ou des croûtes dans les images et/ou dans les images combinées à travers le dispositif de traitement d'images (S7) ;
- calculer la surface proportionnelle des zones cutanées identifiées par segment à travers le moyen d'évaluation (S8) ;
- déterminer un degré de rougeur de la surface cutanée, le degré de croûtes de la surface cutanée et un degré de voûte de la surface cutanée dans les zones cutanées identifiées par segment à travers le dispositif de traitement d'images (S9) ;
- calculer une value de segment chaque fois sur la base du degré de rougeur de la surface cutanée, du degré de croûtes de la surface cutanée, du degré de voûte de la surface cutanée et de la proportion des zones cutanées identifiées de la surface cutanée par segment à travers le moyen d'évaluation (S10) ;
- calculer une valeur totale pondérée sur la base des valeurs de segment et d'un facteur de pondération pour chaque valeur de segment à travers le moyen d'évaluation (S11) ;
- émettre la valeur totale et/ou les valeurs de segment à travers une unité de sortie (16) (S12),
**caractérisé en ce que**
la génération d'images combinées comprend l'identification de zones identiques dans au moins deux images et l'assemblage des images avec un chevauchement dans les zones identifiées.

2. Procédé selon la revendication 1,
**caractérisé en ce que**
les images et/ou les images combinées sont émises sur l'unité de sortie (16).

3. Procédé selon la revendication 2,
**caractérisé en ce que**
les zones cutanées identifiées sont affichées dans les images et/ou dans les images combinées.

4. Procédé selon la revendication 3,
**caractérisé en ce que**
moyennant une entrée à l'unité d'entrée (14), les zones cutanées identifiées peuvent être changées et/ou éliminées et/ou des zones cutanées identifiés supplémentaires peuvent être définies.

5. Procédé selon l'une quelconque des revendications 2 à 4,
**caractérisé en ce que**
les segments sont affichés en particulier dans les images et/ou dans les images combinées.

6. Procédé selon la revendication 5,
**caractérisé en ce que**
les segments peuvent être changés moyennant une entrée de l'unité d'entrée (14).

7. Procédé selon l'une quelconque des revendications 1 à 6,
**caractérisé en ce que**
les régions tête, extrémités supérieures, jambes et torse sont identifiées dans les images et/ou dans les images combinées comme des segments dans la surface cutanée identifiée.

8. Procédé selon l'une quelconque des revendications 1 à 7,
**caractérisé en ce que**
les valeurs de segment et/ou la valeur totale pondérée peuvent/peut être changée(s) moyennant une entrée de l'unité d'entrée (14).

9. Procédé selon l'une quelconque des revendications 1 à 8,
**caractérisé en ce que**
la transition entre des étapes de procédé individuelles résulte en réponse à une entrée de l'unité d'entrée.

10. Procédé selon l'une quelconque des revendications 1 à 9,
**caractérisé en ce que**

la valeur totale pondérée est enregistrée dans le support de stockage.

11. Système (02) de prise de vues du corps entier et de traitement d'images ayant un système d'imagerie (08) pour capturer des images numériques d'un corps à examiner (04) à l'aide d'une pluralité de positions prédéterminées du corps à examiner (04), ledit système d'imagerie (08) présentant au moins un moyen de prise de vues (06) et un moyen d'ajustage (10) automatique, un support de stockage relié avec le système d'imagerie (08), et conçu au moins pour enregistrer des images numériques, ainsi qu'un moyen de calcul (12) comprenant un dispositif de traitement d'images, un moyen d'évaluation, une unité de sortie (16) et une unité d'entrée (14), ledit moyen de calcul étant relié avec le support de stockage,
dans lequel ledit dispositif de traitement d'images est configuré pour générer des images combinées à partir d'au moins deux images différentes de la même position du corps à examiner, ladite génération d'images combinées comprenant l'identification de zones identiques dans au moins deux images et l'assemblage des images avec un chevauchement dans les zones identifiées, pour identifier de la surface cutanée du corps à examiner dans les images et/ou dans les images combinées, pour identifier des segments prédéfinis de la surface cutanée identifiée dans les images et/ou dans les images combinées, pour identifier des zones cutanées qui présentent des rougeurs ou des croûtes dans les images et/ou dans les images combinées ainsi que pour déterminer un degré de rougeur de la surface cutanée, un degré de croûtes de la surface cutanée et un degré de voûte de la surface cutanée dans les zones cutanées identifiées par segment,
et dans lequel le moyen d'évaluation est configuré pour calculer la surface proportionnelle des zones cutanées identifiées par segment, pour calculer une value de segment chaque fois sur la base du degré de rougeur de la surface cutanée, du degré de croûtes de la surface cutanée, du degré de voûte de la surface cutanée et de la proportion de la surface cutanée des zones cutanées identifiées, et pour calculer une valeur totale pondérée sur la base des valeurs de segment et d'un facteur de pondération pour chaque valeur de segment.

12. Système de prise de vues du corps entier et de traitement d'images selon la revendication 11,
**caractérisé en ce que**
le support de stockage est configuré pour enregistrer des données en matière du corps à examiner, des images combinées, des valeurs de segment et des valeurs totales pondérées.

13. Système de prise de vues du corps entier et de traitement d'images selon la revendication 11 ou 12,
**caractérisé en ce que**
le moyen d'ajustage (10) est commandé via un moyen de commande qui commande le moyen de prise de vues en fonction de données en matière du corps à examiner (04) et de la position du corps à examiner (04).

14. Système de prise de vues du corps entier et de traitement d'images selon l'une quelconque des revendications 11 à 13,
**caractérisé en ce que**
l'unité de sortie (16) est conçue comme un écran et/ou comme une imprimante.

Fig. 1

Fig. 2

Fig. 3

Fig. 4

B58

B14

B1

B2

B3

B4

B9

B10

B11

B12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2012064170 A1
- WO 2004095372 A1